Europäisches Patentamt

European Patent Office

Office européen des brevets

(11) Numéro de publication: **0 320 320 B1**

# FASCICULE DE BREVET EUROPEEN

(45) Date de publication de fascicule du brevet: **23.12.92**

(51) Int. Cl.5: **A61K 9/20**, A61K 9/22, A23K 1/18

(21) Numéro de dépôt: **88402677.4**

(22) Date de dépôt: **24.10.88**

---

(54) **Comprimé pour animal domestique.**

---

(30) Priorité: **08.12.87 FR 8717068**

(43) Date de publication de la demande:
**14.06.89 Bulletin 89/24**

(45) Mention de la délivrance du brevet:
**23.12.92 Bulletin 92/52**

(84) Etats contractants désignés:
**AT BE CH DE ES GB GR IT LI LU NL SE**

(56) Documents cités:
**EP-A- 0 282 020**
**FR-A- 1 568 930**
**US-A- 3 608 064**

**CHEMICAL ABSTRACTS, vol. 85, no. 12, 20 septembre 1976, abstract no. 83225f, Columbus, Ohio, US**

(73) Titulaire: **SO.GE.VAL S.A.**
**200 Route de Mayenne**
**Laval Mayenne(FR)**

(72) Inventeur: **Daoudal, José**
**39 rue Benoît Frachon**
**Laval Mayenne(FR)**

(74) Mandataire: **Cabinet Pierre HERRBURGER**
**115, Boulevard Haussmann**
**F-75008 Paris(FR)**

---

Rank Xerox (UK) Business Services

## Description

La présente invention concerne un comprimé permettant de faciliter l'absorption de substances médicamenteuses par des animaux domestiques, notamment des chiens ou des chats.

Le nombre des animaux domestiques tels que chiens et chats est en augmentation constante. Parallèlement à cette augmentation, on assiste à une évolution des soins apportés à ces animaux.

Il faut constater qu'à ce jour, le suivi des traitements administrés n'est pas toujours bien assuré, en raison de la difficulté à faire absorber les médications à l'animal.

En effet, de nombreuses substances de traitement ou de prévention des maladies et des déséquilibres organiques exercent un effet répulsif pour l'odorat de ces animaux qui refusent des les absorber.

Or, jusqu'à présent, aucune solution n'avait encore été proposée pour résoudre ce problème.

La présente invention a pour objet de combler cette lacune en position un comprimé pour animal domestique, notamment pour chien ou pour chat susceptible d'être absorbé spontanément par l'animal.

Conformément à l'invention, ce comprimé est caractérisé en ce qu'il est constitué par au moins un noyau contenant un ou plusieurs principes actifs, exerçant un effet répulsif pour l'odorat de l'animal, totalement englobés dans une matrice appétente renfermant un mélange de poudre de foie et de levure de bière.

En travaillant les facteurs d'appétence pour chiens et chats, on s'est aperçu, de manière surprenante que la poudre de foie est particulièrement prisée par ces animaux et qu'un mélange poudre de foie/ levure de bière est de nature à faciliter dans une large mesure l'absorption des médicaments par les chiens et les chats permettant, par suite, de leur administrer facilement des traitements préventifs ou curatifs.

A titre d'exemple, il convient de mentionner l'existence du document CHEMICAL ABSTRACTS, vol.85, n° 12, 20/09/76, abstract n° 83 225f qui décrit un vermifuge de composition particulière destiné aussi bien à la médecine humaine que vétérinaire et présenté en comprimés contenant, en plus du principe actif, des additifs tels que de la poudre de foie de boeuf ou du sucrose.

Il s'agit cependant ici d'un comprimé classique qui est bien décrit comme ayant un goût agréable, mais dans lequel le principe actif n'est pas englobé dans une matrice appétente.

Dans ces conditions, les qualités gustatives susmentionnées ne peuvent être garanties que dans l'hypothèse dans laquelle le principe actif n'a pas un caractère désagréable trop marqué ou demeure en faible quantité ; il est à cet effet instructif de noter que la préparation décrite dans le document considéré ne contient que 110 mg de substances actives pour un poids total de 2 500 mg, ce qui est extrêmement peu.

En fait, seule la présente invention, conformément à laquelle les principes actifs sont placés dans un noyau central enrobé d'une couche appétente permet la préparation de comprimés à charge élevée en principes actifs, la couche externe appétente masquant totalement l'odeur du médicament, répulsive pour l'animal.

Selon une autre caractéristique de l'invention, la matrice appétente renferme un excipient de compression et/ou des agents lubrifiants.

Cet excipient peut notamment être choisi dans le groupe formé par la cellulose, le lactose et le phosphate bicalcique, les amidons de blé et de maïs.

Le rôle de cet excipient est de faciliter la fabrication du comprimé.

Les agents lubrifiants peuvent notamment être choisis dans le groupe formé par les stéarates, le talc, etc ...

Bien entendu, le ou les noyaux du comprimé conforme à l'invention peuvent renfermer différents types de principes actifs administrables par voie orale et choisis dans toutes les classes thérapeutiques et diététiques existantes et à venir, parmi lesquelles on peut mentionner les exemples suivants :

Exemples de classes thérapeutiques :

- Anti-infectieux (antibiotiques, sulfamides ...),
- Anti-parasitaires internes et externes,
- Anti-inflammatoires et anti-histaminiques,
- Vaccins oraux,
- Hormones,
- Substances de thérapeutique digestive, tels les pansements et sédatifs gastro intestinaux, les florez de remplacement, les antidiarrhéiques, les hépato-protecteurs, les antispasmodiques, les laxatifs, les antiseptiques intestinaux, les réhydratants oraux ...,
- Substances de thérapeutique cardio-vasculaire tels les analeptiques cardiaques et cardiovasculaires, les hémostatiques, les vasoconstricteurs et dilatateurs ...,
- Substances de thérapeutique respiratoire tels les analeptiques respiratoires, les antitussifs, les bronchodilatateurs, les bronchosécrétolytiques, les antiseptiques respiratoires,
- Substances agissant sur le système nerveux : analgésiques, sédatifs et tranquillisants, antiépileptiques, anesthésiques, orexigènes et anorexigènes,
- Substances de thérapeutique immunitaire tels les immunodépresseurs, les immunostimulants, les immunoglobulines de remplacement ...,
- Diurétiques,
- Substances anticancéreuses tels les antimitotiques,

Exemples de classes diététiques et thérapeutiques :

- Les macro et oligo-éléments,
- Les vitamines,
- Les acides aminés et les protéines,
- Les acides gras,
- Les glucides,
- Les extraits de plantes ou d'organes d'animaux.

Les caractéristiques du comprimé pour animal domestique qui fait l'objet de l'invention seront décrites plus en détail en se référant aux dessins annexés, dans lesquels :
- la figure 1 est une coupe schématique d'un comprimé ayant un seul noyau pouvant contenir plusieurs principes actifs,
- la figure 2 est une coupe schématique d'un comprimé ayant plusieurs noyaux pouvant contenir plusieurs principes actifs.

Selon la figure, le comprimé est constitué par un noyau 1 totalement revêtu d'une matrice 2 particulièrement appétente pour l'animal.

Le noyau 1 contient un ou plusieurs principes actifs quelconques tels que défini ci-dessus.

La matrice 2 est constituée par un mélange de poudre de foie, de levure de bière et d'un excipient de compression tel que cellulose, lactose, phosphate bicalcique, amidon de blé ou amidon de maïs, agents lubrifiants tels que talc, stéarates ...

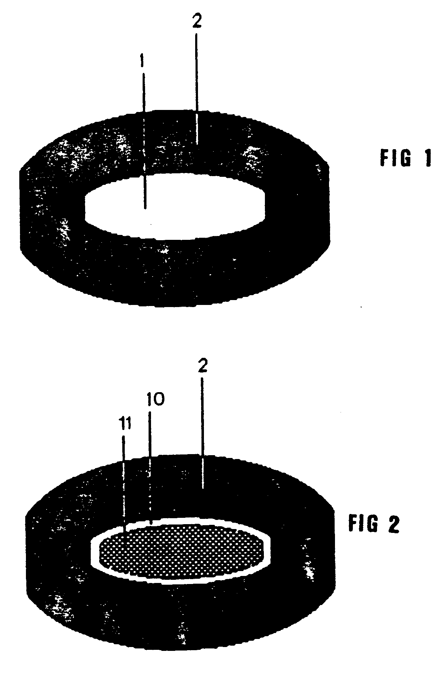

Selon la figure 2, le noyau 1 se subdivise en une partie centrale 11 contenant un ou plusieurs principes actifs et une partie périphérique 10 contenant un autre principe actif.

Il a été prouvé qu'un tel comprimé pouvait être absorbé sans difficulté par les chiens ou les chats.

## Revendications

1. Comprimé permettant de faciliter l'absorption de substances médicamenteuses par des animaux domestiques, notamment des chiens ou des chats, caractérisé en ce qu'il est constitué par au moins un noyau (1) contenant un ou plusieurs principes actifs exerçant un effet répulsif pour l'odorat de l'animal totalement englobé dans une matrice appétente (2) renfermant un mélange de poudre de foie et de levure de bière.

2. Comprimé selon la revendication 1, caractérisé en ce que la matrice appétente (2) renferme un excipient de compression.

3. Comprimé selon la revendication 2, caractérisé en ce que l'excipient de compression est choisi dans le groupe formé par la cellulose, le lactose, le phosphate bicalcique, les amidons.

4. Comprimé selon l'une quelconque des revendications 1 à 3, caractérisé en ce que la matrice appétente renferme un ou des agents lubrifiants.

5. Comprimé selon l'une quelconque des revendications 1 à 4, caractérisé en ce que les agents lubrifiants sont choisis dans le groupe formé du talc et des stéarates.

## Claims

1. Tablet enabling the taking of medicinal substances by domestic animals, in particular dogs or cats, to be facilitated, characterised in that it consists of at least one core (1), containing one or more active principles which exert a repellent effect on the animal's sense of smell, completely enclosed in an appealing matrix (2) containing a mixture of liver powder and brewer's yeast.

2. Tablet according to Claim 1, characterised in that the appealing matrix (2) contains a tableting excipient.

3. Tablet according to Claim 2, characterised in that the tableting excipient is chosen from the group composed of cellulose, lactose, dicalcium phosphate and starches.

4. Tablet according to any one of Claims 1 to 3, characterised in that the appealing matrix contains one or more lubricants.

5. Tablet according to any one of Claims 1 to 4, characterised in that the lubricants are chosen from the group composed of talc and stearates.

## Patentansprüche

1. Tablette zum Vereinfachen der Aufnahme (Absorption) medikamentöser Substanzen durch Haustiere, insbesondere durch Hunde oder durch Katzen, dadurch gekennzeichnet, daß sie aus mindestens einem Kern (1) besteht, der einen oder mehrere aktive/n, für den Geruchssinn des Tieres einen abstoßenden Effekt ausübende/n Bestandteil/e enthält und völlig in einer instinktmäßig begehrten Stammasse (2), die eine Mischung aus Leberpulver und Bierhefe enthält, zusammengefaßt oder eingehüllt ist.

2. Tablette nach Anspruch 1, dadurch gekennzeichnet, daß die instinktmäßig begehrte Stammasse (2) ein Druckbindemittel enthält.

3. Tablette nach Anspruch 2, dadurch gekennzeichnet, daß Druckbindemittel aus der Gruppe ausgewählt ist, die aus Zellulose, Lactose, Dicalciumphosphat, Stärke, gebildet ist.

4. Tablette nach irgendeinem der Ansprüche 1 bis 3, dadurch gekennzeichnet, daß die instinktmäßig begehrte Stammmasse ein oder mehrere Schmiermittel enthält.

5. Tablette nach irgendeinem dar Ansprüche 1 bis 4, dadurch gekennzeichnet, daß die Schmiermittel aus der Gruppe ausgewählt sind, die aus Talk und Stearaten gebildet ist.

FIG 1

FIG 2